(19) Europäisches Patentamt
European Patent Office
Office européen des brevets

(11) Veröffentlichungsnummer: **0 269 563 B1**

(12) **EUROPÄISCHE PATENTSCHRIFT**

(45) Veröffentlichungstag der Patentschrift: **06.11.91**

(51) Int. Cl.5: **C09B 1/20**

(21) Anmeldenummer: **87810608.7**

(22) Anmeldetag: **22.10.87**

(54) **Verfahren zur Herstellung von 1-Aminoanthrachinonen.**

(30) Priorität: **28.10.86 CH 4267/86**

(43) Veröffentlichungstag der Anmeldung:
**01.06.88 Patentblatt 88/22**

(45) Bekanntmachung des Hinweises auf die Patenterteilung:
**06.11.91 Patentblatt 91/45**

(84) Benannte Vertragsstaaten:
**BE CH DE ES FR GB IT LI NL**

(56) Entgegenhaltungen:
**EP-A- 0 014 106**
**FR-A- 2 249 068**

**CHEMICAL ABSTRACTS, Band 86, 1977, Seite 68, Zusammenfassung Nr. 31015p, Columbus, Ohio, US; & JP-A-76 102 022 (KAWASAKI KASEI CHEMICALS LTD) 09-09-1976**

(73) Patentinhaber: **CIBA-GEIGY AG**
**Klybeckstrasse 141**
**CH-4002 Basel(CH)**

(72) Erfinder: **Plattner, Eric, Prof. Dr.**
**Jurastrasse 18**
**CH-4411 Seltisberg(CH)**
Erfinder: **Seifert, Gottfried**
**Mühlemattweg 20**
**CH-4312 Magden(CH)**
Erfinder: **Somlo, Tibor, Dr.**
**Florastrasse 8**
**CH-4127 Birsfelden(CH)**

**EP 0 269 563 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 1-Aminoanthrachinonen aus 5-Nitro-1,4,4a,9a-tetrahydroanthrachinonen.

1-Aminoanthrachinone sind ein wichtiges Zwischenprodukt zur Herstellung von Farbstoffen und im Sinne einer wirtschaftlichen Weiterverarbeitung wird die Herstellung sehr reiner 1-Aminoanthrachinone angestrebt.

Aus der DE-A- 2 450 883 ist ein Verfahren zur Herstellung von 1-substituierten Anthrachinonen bekannt, das ebenfalls von 5-Nitro-1,4,4a,9a-tetrahydroanthrachinon ausgeht und dies bei 0 bis 200°C in flüssigem Medium mit einem basischen Reduktionsmittel oder einer Kombination aus einem Reduktionsmittel und einer basischen Verbindung umsetzt. Nach diesem Verfahren können aber nur Produkte erhalten werden, die die gewünschte Reinheit nicht aufweisen.

Aufgabe der Erfindung ist es, ein Verfahren anzugeben, das es erlaubt, 1-Aminoanthrachinone mit grösserer Reinheit herzustellen. Erfindungsgemäss wird diese Aufgabe dadurch gelöst, dass das Verfahren unter Druck innerhalb eines bestimmten Temperaturbereichs ausgeführt wird.

Die Erfindung betrifft somit ein Verfahren zur Herstellung von 1-Aminoanthrachinonen der Formel

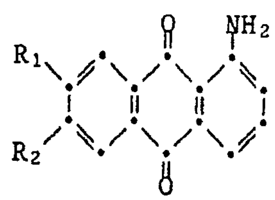

(I)

worin $R_1$ und $R_2$ unabhängig voneinander ein Wasserstoffatom, $C_1$-$C_4$-Alkyl oder halogen bedeuten, durch Umsetzung eines 5-Nitro-1,4,4a,9a-tetrahydroanthrachinons der Formel

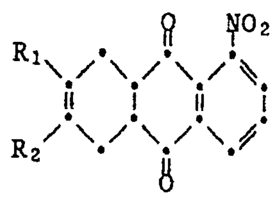

(II)

mit einem basischen Reduktionsmittel bei 110 bis 180°C in einem wässrig-organischen Medium, dadurch gekennzeichnet, dass man die Reaktion unter Druck durchführt, wobei der Druck über dem Sättigungsdampfdruck des wässrig-organischen Mediums bei der gewählten Temperatur liegt.

Das als Ausgangsmaterial verwendete 5-Nitro-1,4,4a,9a-tetrahydroanthrachinon der Formel (II) ist bekannt und lässt sich z.B. durch Diels-Alder-Reakton von Butadien mit 5-Nitro-1,4-naphthochinon herstellen. Das 5-Nitro-1,4-naphthochinon ist z.B. durch elektrolytische Oxidation von 1-Nitronaphthalin oder Nitrierung von 1,4-Napththochinon zugänglich.

Beispiele für Ausgangsmaterialien der Formel (II) sind: 5-Nitro-1.4.4a.9a-tetrahydroanthrachinon, 2- oder 3-Methyl-5-nitro-1.4.4a.9a-tetrahydroanthrachinon, 2- oder 3-Chlor-5-nitro-1.4.4a.9a-tetrahydroanthrachinon und 2.3-Dimethyl-5-nitro-1.4.4a.9a-tetrahydroanthrachinon. Unter diesen Verbindungen ist 5-Nitro-1.4.4a.95-tetrahydroanthrachinon als Ausgangsmaterial zur Herstellung des wichtigen Farbstoffzwischenproduktes 1-Aminoanthrachinon bevorzugt.

Als basische Reduktionsmittel können alle Verbindungen verwendet werden, die 5-Nitroverbindungen der Formel (II) zu den 1-Aminoverbindungen der Formel (I) im wässrig-organischen Medium unter Druck reduzieren können. Beispiele für solche Reduktionsmittel sind vor allem Hydrogensulfide (wie Lithiumhydrogensulfid, Natriumhydrogensulfid, Kaliumhydrogensulfid, Rubidiumhydrogensulfid und Ammoniumhydrogensulfid), Sulfide (wie Lithiumsulfid, Natriumsulfid, Kaliumsulfid, Cäsiumsulfid und Ammoniumsulfid) Disulfide und Polysulfide (wie Natriumdisulfid, Natriumpolysulfid, Kaliumdisulfid, Kaliumpolysulfid, Ammoniumdisulfid und Ammoniumpolysulfide).

Das basische Reduktionsmittel wird in Mengen von mindestens 0,4 Mol, vorzugsweise 0,4 bis 2,0 Mol und insbesondere 0,5 Mol pro Mol 5-Nitroverbindung der Formel (II) eingesetzt.

Die Durchführung der Reaktion erfolgt in einem wässrig-organischen Medium, dessen organischer Anteil ein mit Wasser nicht mischbares organisches Lösungsmittel darstellt, in welchem die Produkte eine ausreichende Löslichkeit besitzen. Solche Lösungsmittel sind beispielsweise Aromaten wie Benzol und vorzugweise alkylierte und halogenierte Benzole wie, Toluol, Xylol, Monochlorbenzol, Dichlorbenzol, ferner Methylisobutylketon sowie n-Butanol, 2-Butanol und iso-Butanol und Gemische davon. Bevorzugt wird die Durchführung des Verfahrens in Wasser/Toluol als Reaktionsmedium.

Die Reaktionstemperatur liegt im Bereich von 110 bis 180°C und insbesondere bei 155 bis 165°C. Die Reaktionszeit bei diskontinuierlicher Arbeitsweise beträgt ca. 10 bis 30 Minuten, während für die kontinuierliche Reaktionsführung Verweilzeiten von etwa 2 bis 5 Minuten erforderlich sind.

Erfindungsgemäss wird unter Druck gearbeitet, wobei der Druck stets über dem Sättigungsdampfdruck des wässrig-organischen Mediums bei der gewählten Reaktionstemperatur liegen muss. Der Druck liegt in der Regel zwischen 1,5 und 20 bar, d.h. man arbeitet üblicherweise mit 0,5 bis 2,0 bar über dem Sättigungsdampfdruck.

Die erfindungsgemässe Reaktionsführung unter Druck bietet gegenüber der drucklosen Verfahrensweise eine Reihe wesentlicher Vorteile. Beispielsweise wird eine sehr saubere Trennung zwischen organischer und wässriger Phase erreicht, wobei die organische Phase im wesentlichen die Gesamtmenge an Produkt ohne Nebenprodukte enthält. Diese bleiben in der wässrigen Phase gelöst, welche unter Druck abgetrennt wird. Dies ist die Grundlage für deutlich verbesserte Kristallisationsbedingungen für 1-Aminoanthrachinone. Die erfindungsgemäss hergestellten 1-Aminoanthrachinone fallen gut kristallisiert in 99 %iger Reinheit an (praktisch ohne Einschlüsse von Verunreinigungen), und die Ausbeute beträgt über 98 %. 1-Aminoanthrachinone dieser Qualität lassen sich direkt, d.h. ohne Reinigungsoperationen, zur Farbstoffherstellung verwenden.

Bedingt durch die saubere Phasentrennung gestattet das erfindungsgemässe Verfahren auch eine kontinuierliche Arbeitsweise.

Ferner kann bei einer höheren Reaktionstemperatur gearbeitet werden, wodurch Hydroxylamine, welche als zwischenprodukte gebildet werden, praktisch vollständig in die entsprechenden Aminoverbindungen überführt werden. Auf Grund der höheren Reaktionstemperatur und der damit verbundenen grösseren Löslichkeit der Produkte in der organischen Phase wird auch eine Verringerung der Menge an organischer Phase erreicht, was im Hinblick auf ein industrielles Verfahren von grosser Bedeutung ist.

Die technische Durchführung des erfindungsgemässen Verfahrens erfolgt beispielsweise so, dass man eine Suspension der 5-Nitroverbindung der Formel (II) in Toluol herstellt und einer wässrigen Natriumhydrogensulfidlösung zudosiert. Anschliessend wird das Phasengemisch in einen Autoklaven überführt, verschlossen und während 20 Minuten erhitzt. Sodann wird die wässrige Phase unter Druck abgetrennt, die organische Phase gekühlt und daraus die 1-Aminoverbindung der Formel (I) durch Filtration isoliert.

Die folgenden Beispiele erläutern die Erfindung. Teile bedeuten Gewichtsteile.

Beispiel 1:

In einem 2 1 Autoklaven mit Ankerrührer werden 64,3 Teile 5-Nitro-1,4,4a,9a-tetrahydro-anthrachinon, 750 Teile Toluol, 250 Teile Wasser und 8,8 Teile Natriumhydrogensulfid vorgelegt. Das Phasengemisch wird unter Rühren auf 160°C bei einem Ueberdruck von 10 bis 11 bar geheizt. Dann wird der Rührer abgestellt und die untere Schicht unter diesem Druck abgetrennt. Die obere Phase wird auf Raumtemperatur gekühlt, wobei das reine 1-Aminoanthrachinon auskristallisiert. Es wird durch Filtration isoliert, mit 75 Teilen Toluol gewaschen und im Vakuum bei 80°C getrocknet. Bei den weiteren Ansätzen werden der grösste Teil (z.B. 3/4) der Mutterlauge anstelle von neuem Toluol eingesetzt. Auf diese Weise werden 55 Teile 1-Aminoanthrachinon mit einer Reinheit von 99 % erhalten. Das entspricht einer Ausbeute von etwa 99 % der Theorie.

Beispiel 2:

In dem 2 1 Autoklaven werden 64,3 Teile 5-Nitro1,4,4a,9a-tetrahydro-anthrachinon und 750 Teile Toluol vorgelegt. Das Gemisch wird im geschlossenen System auf 160°C geheizt und bei dieser Temperatur eine Lösung von 8,8 Teilen NaHS in 250 Teilen Wasser innerhalb 5 Minuten zudosiert. Der Druck beträgt 10 bar. Die Aufarbeitung erfolgt analog Beispiel 1. Es wird dieselbe Ausbeute und Qualität wie im Beispiel 1 erreicht.

Beispiel 3: (Kontinuierliche Fahrweise)

Eine Lösung von 100 Teilen 5-Nitro-1,4,4a,9a-tetrahydro-anthrachinon in 400 Teilen Toluol, 1000 Teilen toluolische Mutterlauge und eine Lösung von 8,8 Teilen Natriumhydrogensulfid in 400 Teilen Wasser werden gleichzeitig in einen Rohrreaktor (Volumen 100 ml) mit statischen Mischelementen zudosiert. Die mittlere Verweilzeit beträgt ca. 3 Minuten. Die Eingangstemperatur der Komponenten wird so geregelt, dass das Reaktionsgemisch den Reaktor mit 160°C verlässt und in einem Trenngefäss unter Druck (10 bar) kontinuierlich in eine wässerige und organische Phase getrennt wird. Die organische Phase wird im Aufarbeitungsreaktor entspannt und auf Raumtemperatur gekühlt. Dabei kristallisiert das 1-Aminoanthrachinon aus. Es wird filtriert, mit Toluol gewaschen und im Vakuum bei 80°C getrocknet. Mit der toluolischen Mutterlauge wird die wässerige Phase nachextrahiert und teilweise in den Reaktor zurückgeführt. Im stationären Betrieb werden pro Stunde 86 Teile 1-Aminoanthrachinon mit einer Reinheit von 99 % (LC) erhalten. Das sind 98 % der Theorie bezogen auf 5-Nitro-tetrahydroanthrachinon.

Beispiel 4:

Werden im Beispiel 1 anstatt 64,3 Teile 5-Nitro-1,4,4a,9a-tetrahydro-anthrachinon 71,3 Teile 5-Nitro-2,3-dimethyl-1,4,4a,9a-tetrahydro-anthrachinon (hergestellt aus 1-Nitronaphthochinon und 2,3-Dimethyl-1,4-butadien durch Diels-Alder-Reaktion) eingesetzt, so erhält man durch die gleiche Arbeitsweise 61,9 Teile 1-Amino-6,7-dimethyl-anthrachinon mit einem Gehalt von 99 %. Das entspricht einer Ausbeute von 99 % der Theorie.

**Patentansprüche**

1. Verfahren zur Herstellung von 1-Aminoanthrachinon der Formel

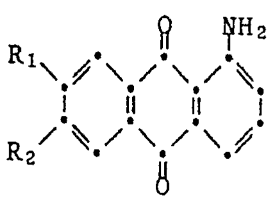

(I)

worin $R_1$ und $R_2$ unabhängig voneinander Wasserstoff, $C_1$-$C_4$-Alkyl oder Halogen bedeuten, durch Umsetzung eines 5-Nitro-1,4,4a,9a-tetrahydroanthrachinons der Formel

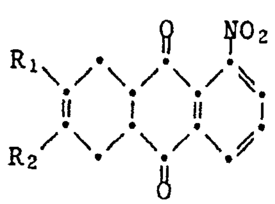

(II)

mit einem basischen Reduktionsmittel, bei 110 bis 180°C in einem wässrig-organischen Medium, dadurch gekennzeichnet, dass man die Reaktion unter Druck durchführt, wobei der Druck über dem Sättigungsdampfdruck des wässrig-organischen Mediums bei der gewählten Temperatur liegt.

2. Verfahren gemäss Anspruch 1, dadurch gekennzeichnet, dass die Reaktion bei 155 bis 165°C durchgeführt wird.

3. Verfahren gemäss Anspruch 1, dadurch gekennzeichnet, dass der organische Anteil des wässrig-organischen Mediums ein mit Wasser nicht mischbares Lösungsmittel ist.

4. Verfahren gemäss Anspruch 3, dadurch gekennzeichnet, dass die Reaktion in Wasser/Toluol durchgeführt wird.

## Claims

1. A process for the preparation of a 1-aminoanthraquinone of the formula

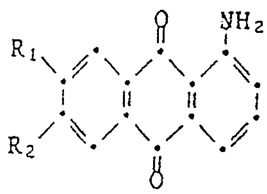

(I)

in which $R_1$ and $R_2$ are each independently of the other hydrogen, $C_1$-$C_4$alkyl or halogen, by reacting a 5-nitro-1,4,4a,9a-tetrahydro-anthraquinone of the formula

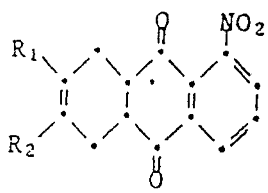

(II),

with a basic reducing agent, at from 110 to 180 °C, in an aqueous-organic medium, which process comprises carrying out the reaction under pressure, where the pressure is above the saturation vapour pressure of the aqueous-organic medium at the chosen temperature.

2. A process according to claim 1, wherein the reaction is carried out at from 155 to 165 °C.

3. A process according to claim 1, wherein the organic component of the aqueous-organic medium is a water-immiscible solvent.

4. A process according to claim 3, wherein the reaction is carried out in water/toluene.

## Revendications

1. Procédé de préparation de 1-amino-anthraquinone de formule

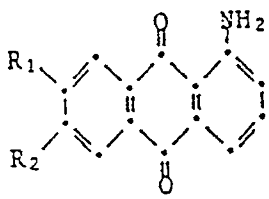

(I)

dans laquelle $R_1$ et $R_2$ représentent, indépendamment l'un de l'autre, un atome d'hydrogène ou d'halogène ou un groupe alkyle en $C_1$-$C_4$,
par réaction d'une 5-nitro-1,4,4a,9a-tétrahydro-anthraquinone de formule

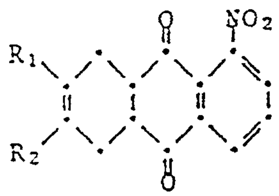

(II)

avec un réducteur basique, à 110-180°C, dans un milieu aqueux-organique, caractérisé en ce que l'on effectue la réaction sous pression, la pression étant supérieure à la pression de vapeur saturante du milieu aqueuxorganique à la température choisie.

2. Procédé conforme à la revendication 1, caractérisé en ce que l'on effectue la réaction entre 155 et 165°C.

3. Procédé conforme à la revendication 1, caractérisé en ce que le constituant organique du milieu aqueux-organique est un solvant non miscible à l'eau.

4. Procédé conforme à la revendication 3, caractérisé en ce que la réaction est effectuée dans un mélange d'eau et de toluène.